# EUROPEAN PATENT SPECIFICATION

(11) **EP 1 319 077 B1**
(45) Date of publication and mention of the grant of the patent: **31.08.2005**
(21) Application number: 01983465.4
(22) Date of filing: 06.09.2001
(51) Int. Cl.: C12N 15/54, C12N 15/31, C12N 1/21, C12N 9/12, C12P 13/08, C12Q 1/68

(54) **NUCLEOTIDE SEQUENCES WHICH CODE FOR THE TMK GENE**
NUKLEINSÄUREN KODIEREND FÜR DAS TMK GEN
SEQUENCES NUCLEOTIDIQUES CODANT POUR LE GENE TMK

(30) Priority: 19.09.2000 DE 10046235; 16.08.2001 DE 10140095
(43) Date of publication of application: 18.06.2003
(73) Proprietor: Degussa AG, 40474 Düsseldorf (DE)
(72) Inventor: FARWICK, Mike, 33615 Bielefeld (DE); HUTHMACHER, Klaus, 63584 Gelnhausen (DE); MARX, Achim, 33790 Halle (DE); PFEFFERLE, Walter, 33790 Halle (Westf.) (DE)
(86) International application number: PCT/EP2001/010268
(87) International publication number: WO 2002/024716

(56) References cited:
- WO-A-01/00843
- DATABASE GENSEQ [Online] Derwent; 26 September 2001 (2001-09-26) "Seq Id no 4339 from EP1108790" Database accession no. AAG90585 XP002210352 -& EP 1 108 790 A (KYOWA HAKKO KOGYO KK) 20 June 2001 (2001-06-20)
- DATABASE GENSEQ [Online] Derwent; Seq Id 839 from EP1108790, 26 September 2001 (2001-09-26) Database accession no. AAH65804 XP002210353 -& EP 1 108 790 A (KYOWA HAKKO KK) 20 June 2001 (2001-06-20)
- SAHM H ET AL: "CONSTRUCTION OF L-LYSINE, L-THREONINE-, OR L-ISOLEUCINE-OVERPRODUCING STRAINS OF CORYNEBACTERIUM GLUTAMICUM" ANNALS OF THE NEW YORK ACADEMY OF SCIENCES, NEW YORK ACADEMY OF SCIENCES, NEW YORK, NY, US, vol. 782, 1996, pages 25-39, XP000943151 ISSN: 0077-8923
- EIKMANNS B J ET AL: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK, DORDRECHT, NL, vol. 64, no. 2, 1993, pages 145-163, XP000918559
- LAVIE ARNON ET AL: "Structural basis for efficient phosphorylation of 3'-azidothymidine monophosphate by Escherichia coli thymidylate kinase." PROCEEDINGS OF THE NATIONAL ACADEMY OF SCIENCES OF THE UNITED STATES, vol. 95, no. 24, 24 November 1998 (1998-11-24), pages 14045-14050, XP002210351 Nov. 24, 1998 ISSN: 0027-8424

## Description

### Field of the Invention

The invention provides isolated coryneform bacteria, wherein the expression of nucleotide sequences from coryneform bacteria which code for the tmk gene is eliminated and a process for the fermentative preparation of L-lysine using bacteria in which the tmk gene is eliminated.

### Prior Art

L-Amino acids, in particular L-lysine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and which produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

### Object of the Invention

The inventors had the object of providing new measures for improved fermentative preparation of L-lysine.

### Summary of the Invention

When L-lysine or lysine are mentioned in the following, not only the bases but also the salts, such as e.g. lysine monohydrochloride or lysine sulfate, are meant by this.

An isolated polynucleotide from coryneform bacteria, comprising a polynucleotide sequence which codes for the tmk gene, chosen from the group consisting of
a) polynucleotide which codes for a polypeptide which has the amino acid sequence of SEQ ID No. 2,
b) polynucleotide which codes for a polypeptide which comprises an amino acid sequence which is identical to the extent of at least 90% to the amino acid sequence of SEQ ID No. 2,
c) polynucleotide which is complementary to the polynucleotides of a) or b), is described.

The polypeptide has the activity of thymidylate kinase.

The description also contains:
a polynucleotide, in particular DNA, which is capable of replication and has the nucleotide sequence as shown in SEQ ID No.1;
a polynucleotide which codes for a polypeptide which has the amino acid sequence as shown in SEQ ID No. 2.

There are claimed:
a vector pXK99Etmk containing parts of the described polynucleotide with SEQ ID NO:1,
and coryneform bacteria, in which the tmk gene is eliminated by an insertion or deletion.

The invention provides:
1. Isolated coryneform bacteria wherein the activity or concentration of the protein having an amino acid sequence which has at least 90 % identity to the amino acid sequence set out in SEQ ID NO:2 and has thymidylate kinase activity is reduced to 0 % of the activity or concentration of said protein of the starting microorganism.
2. Coryneform bacteria according to claim 1, wherein the amino acid sequence of said protein is set out in SEQ ID NO:2.
3. Isolated coryneform bacteria wherein the activity or concentration of the protein encoded by the polynucleotide set out in SEQ ID NO:1 is reduced to 0 % of the activity or concentration of the activity or concentration of the protein of the starting microorganism.

The description also provides polynucleotides which substantially comprise a polynucleotide sequence, which are obtainable by screening by means of hybridization of a corresponding gene library of a coryneform bacterium, which comprises the complete gene or parts thereof, with a probe which comprises the sequence of the polynucleotide described by the invention according to SEQ ID No.1 or a fragment thereof, and isolation of the polynucleotide sequence mentioned.

### Detailed Description of the Invention

Polynucleotides which comprise the sequences described by the invention are suitable as hybridization probes for RNA, cDNA and DNA, in order to isolate, in the full length, nucleic acids or polynucleotides or genes which code for thymidylate kinase or to isolate those nucleic acids or polynucleotides or genes which have a high similarity with the sequence of the tmk gene. They are also suitable for incorporation into so-called "arrays", "micro arrays" or "DNA chips" in order to detect and to determine the corresponding polynucleotides.

Polynucleotides which comprise the sequences according to the description are furthermore suitable as primers with the aid of which DNA of genes which code for thymidylate kinase can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides which have a length of at least 31, 32, 33, 34, 35, 36, 37, 38, 39 or 40 or at least 41, 42, 43, 44, 45, 46, 47, 48, 49 or 50 nucleotides are also suitable. Oligonucleotides with a length of at least 100, 150, 200, 250 or 300 nucleotides are optionally also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the description include a polynucleotide according to SEQ ID No. 1 or a fragment prepared therefrom.

"Polypeptides" are understood as meaning peptides or proteins which comprise two or more amino acids bonded via peptide bonds.

The polypeptides according to the description include a polypeptide according to SEQ ID No. 2 with the biological activity of thymidylate kinase, and also those which are 90% and preferably at least 91%, 93%, 95%, 97% or 99% identical to the polypeptide according to SEQ ID No. 2 and have the activity mentioned.

The invention furthermore relates to a process for the fermentative preparation of L-lysine using coryneform bacteria which in particular already produce L-lysine and in which the nucleotide sequences which code for the tmk gene are eliminated.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 % of the activity or concentration of the wild-type protein or of the activity or concentration of the protein in the starting microorganism.

The microorganisms to which the present invention relates can prepare amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
and L-amino acid-producing mutants or strains prepared therefrom.

The tmk gene from C. glutamicum which codes for thymidylate kinase (EC 2.7.4.9) has been isolated.

To isolate the tmk gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie [Genes and Clones, An Introduction to Genetic Engineering] (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495-508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326)) (1992)) in turn describe a gene library of C. glutamicum ATCC13032 using the cosmid pHC79 (Hohn and Collins, 1980, Gene 11, 291-298).

To prepare a gene library of C. glutamicum in E. coli it is also possible to use plasmids such as pBR322 (Bolivar, 1979, Life Sciences, 25, 807-818) or pUC9 (Vieira et al., 1982, Gene, 19:259-268). Suitable hosts are, in particular, those E. coli strains which are restriction- and recombination-defective, such as, for example, the strain DH5αmcr, which has been described by Grant et al. (Proceedings of the National Academy of Sciences USA, 87 (1990) 4645-4649). The long DNA fragments cloned with the aid of cosmids or other λ vectors can then in turn be subcloned and subsequently sequenced in the usual vectors which are suitable for DNA sequencing, such as is described e.g. by Sanger et al. (Proceedings of the National Academy of Sciences of the United States of America, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The DNA sequence of C. glutamicum which codes for the tmk gene and which, as SEQ ID No. 1, is a constituent of the present description has been found in this manner. The amino acid sequence of the corresponding protein has furthermore been derived from the present DNA sequence by the methods described above. The resulting amino acid sequence of the tmk gene product is shown in SEQ ID No. 2.

Coding DNA sequences which result from SEQ ID No. 1 by the degeneracy of the genetic code are also a constituent of the description. In the same way, DNA sequences which hybridize with SEQ ID No. 1 or parts of SEQ ID No. 1 are a constituent of the description. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention.

In the same way, DNA sequences which hybridize with SEQ ID No. 1 or parts of SEQ ID No. 1 are a constituent of the description. Finally, DNA sequences which are prepared by the polymerase chain reaction (PCR) using primers which result from SEQ ID No. 1 are a constituent of the description. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50ºC - 68ºC, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50ºC - 68ºC being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50ºC to 68ºC in steps of approx. 1 - 2ºC. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait:
Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce L-lysine in an improved manner after elimination of the tmk gene.

To achieve an attenuation, either the expression of the tmk gene or the catalytic properties of the enzyme protein can be eliminated. The two measures can optionally be combined.

The reduction in gene expression can take place by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1996)), Vasicova et al. (Journal of Bacteriology 181: 6188 (1999)) and in known textbooks of genetics and molecular biology, such as e.g. the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum:
Aufhebung der allosterischen Regulation und Struktur des Enzyms [Threonine dehydratase from Corynebacterium glutamicum: Canceling the allosteric regulation and structure of the enzyme]", Reports from the Jülich Research Center, Jül-2906, ISSN09442952, Jülich, Germany, 1994). Summarizing descriptions can be found in known textbooks of genetics and molecular biology, such as e.g. that by Hagemann ("Allgemeine Genetik [General Genetics]", Gustav Fischer Verlag, Stuttgart, 1986).

Possible mutations are transitions, transversions, insertions and deletions. Depending on the effect of the amino acid exchange on the enzyme activity, "missense mutations" or "nonsense mutations" are referred to. Insertions or deletions of at least one base pair (bp) in a gene lead to frame shift mutations, as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stuttgart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986).

A common method of mutating genes of C. glutamicum is the method of "gene disruption" and "gene replacement" described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 (1992)), pGEM-T (Promega Corporation, Madison, WI, USA), pCR2.1-TOPO (Shuman (1994). Journal of Biological Chemistry 269:32678-84; US Patent 5,487,993), pCR®Blunt (Invitrogen, , Groningen, Holland; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of C. glutamicum.

In the method of "gene replacement", a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch et al. (Microbiology 144, 915 - 927 (1998)) to eliminate the pyc gene of C. glutamicum by a deletion.

A deletion, insertion or a base exchange can be incorporated into the tmk gene in this manner.

In addition, it may be advantageous for the production of L-amino acids to enhance, in particular over-express, one or more enzymes of the particular biosynthesis pathway, of glycolysis, of anaplerosis, of the citric acid cycle, of the pentose phosphate cycle, of amino acid export and optionally regulatory proteins, in addition to the attenuation of the tmk gene.

The term "enhancement" in this connection describes the increase in the intracellular activity of one or more enzymes (proteins) in a microorganism which are coded by the corresponding DNA, for example by increasing the number of copies of the gene or genes, using a potent promoter or using a gene or allele which codes for a corresponding enzyme (protein) having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

Thus, for example, for the preparation of L-amino acids, in addition to the elimination of the tmk gene at the same time one or more of the genes chosen from the group consisting of
- the dapA gene which codes for dihydrodipicolinate synthase (EP-B 0 197 335),
- the gap gene which codes for glyceraldehyde 3-phosphate dehydrogenase (Eikmanns (1992), Journal of Bacteriology 174: 6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the pyc gene which codes for pyruvate carboxylase (DE-A-198 31 609),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512; EP-B-0387527; EP-A-0699759),
- the lysE gene which codes for lysine export (DE-A-195 48 222),
- the hom gene which codes for homoserine dehydrogenase (EP-A 0131171),
- the ilvA gene which codes for threonine dehydratase (Möckel et al., Journal of Bacteriology (1992) 8065-8072)) or the ilvA(Fbr) allele which codes for a "feed back resistant" threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the zwa1 gene which codes for the zwa1 protein (DE: 19959328.0, DSM 13115)
can be enhanced, in particular over-expressed.

It may furthermore be advantageous for the production of amino acids, in addition to the elimination of the tmk gene, at the same time for one or more of the genes chosen from the group consisting of
- the pck gene which codes for phosphoenol pyruvate carboxykinase (DE 199 50 409.1, DSM 13047),
- the pgi gene which codes for glucose 6-phosphate isomerase (US 09/396,478, DSM 12969),
- the poxB gene which codes for pyruvate oxidase (DE:1995 1975.7, DSM 13114),
- the zwa2 gene which codes for the Zwa2 protein (DE: 19959327.2, DSM 13113)
to be eliminated

In addition to the elimination of the tmk gene it may furthermore be advantageous for the production of amino acids to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of L-amino acids. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/ Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as, for example, soya oil, sunflower oil, groundnut oil and coconut fat, fatty acids, such as, for example, palmitic acid, stearic acid and linoleic acid, alcohols, such as, for example, glycerol and ethanol, and organic acids, such as, for example, acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as, for example, magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as, for example, fatty acid polyglycol esters, can be employed to control the development of foam. Suitable substances having a selective action, such as, for example, antibiotics, can be added to the medium to maintain the stability of plasmids. To maintain aerobic conditions, oxygen or oxygen-containing gas mixtures, such as, for example, air, are introduced into the culture. The temperature of the culture is usually 20ºC to 45ºC, and preferably 25ºC to 40ºC. Culturing is continued until a maximum of the desired product has formed. This target is usually reached within 10 hours to 160 hours.

Methods for the determination of L-amino acids are known from the prior art. The analysis can thus be carried out, for example, as described by Spackman et al. (Analytical Chemistry, 30, (1958), 1190) by anion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of L-lysine.

The following microorganisms were deposited as pure cultures on 31st July 2001 at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli DH5alphamcr/pXK99E (= DH5αmcr/pXK99E) as DSM 14440,
- Escherichia coli DH5alphamcr/pXK99Etmk (= DH5αmcr/pXK99Etmk) as DSM 14439.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual, 1989, Cold Spring Harbour Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from C. glutamicum ATCC 13032

Chromosomal DNA from C. glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987), Proceedings of the National Academy of Sciences, USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extracts (Stratagene, La Jolla, USA, Product Description Gigapack II XL Packing Extract, Code no. 200217).

For infection of the E. coli strain NM554 (Raleigh et al. 1988, Nucleic Acid Res. 16:1563-1575) the cells were taken up in 10 mM MgSO₄ and mixed with an aliquot of the phage suspension. The infection and titering of the cosmid library were carried out as described by Sambrook et al. (1989, Molecular Cloning: A Laboratory Manual, Cold Spring Harbor), the cells being plated out on LB agar (Lennox, 1955, Virology, 1:190) + 100 µg/ml ampicillin. After incubation overnight at 37°C, recombinant individual clones were selected.

### Example 2

### Isolation and sequencing of the tmk gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01) was cleaved with the restriction enzyme BamHI (Amersham Pharmacia, Freiburg, Germany, Product Description BamHI, Product No. 27-0868-04), The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol. Letters, 123:343-7) into the E. coli strain DH5αmcr (Grant, 1990, Proceedings of the National Academy of Sciences, U.S.A., 87:4645-4649). Letters, 123:343-7) and plated out on LB agar (Lennox, 1955, Virology, 1:190) with 50 mg/l zeocin.

The plasmid preparation of the recombinant clones was carried out with the Biorobot 9600 (Product No. 900200, Qiagen, Hilden, Germany). The sequencing was carried out by the dideoxy chain termination method of Sanger et al. (1977, Proceedings of the National Academy of Sciences, U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analyses were prepared with the XNIP program (Staden, 1986, Nucleic Acids Research, 14:217-231). Further analyses were carried out with the "BLAST search programs" (Altschul et al., 1997, Nucleic Acids Research, 25:3389-3402) against the non-redundant databank of the "National Center for Biotechnology Information" (NCBI, Bethesda, MD, USA).

The resulting nucleotide sequence is shown in SEQ ID No. 1. Analysis of the nucleotide sequence showed an open reading frame of 612 bp, which was called the tmk gene. The tmk gene codes for a polypeptide of 203 amino acids.

### Example 3

Preparation of the expression vector pXK99Etmk for IPTG-induced expression of the tmK gene in C. glutamicum

### 3.1 Cloning of the tmk gene

From the strain ATCC 13032, chromosomal DNA [sic] isolated by the method of Eikmanns et al. (Microbiology 140: 1817-1828 (1994)). On the basis of the sequence of the tmk gene known for C. glutamicum from example 2, the following oligonucleotides were chosen for the polymerase chain reaction (see SEQ ID No. 3 and SEQ ID No. 4):
tmk for:
tmk int:

The primers were chosen here so that the amplified fragment contains the incomplete gene, starting with the native ribosome binding site without the promoter region, and the front region of the tmk gene. Furthermore, the primer tmk for contains the sequence for the cleavage site of the restriction endonuclease Kpn1, and the primer tmk int the cleavage site of the restriction endonuclease XbaI, which are marked by underlining in the nucleotide sequence shown above.

The primers shown were synthesized by MWG-Biotech AG (Ebersberg, Germany) and the PCR reaction was carried out by the standard PCR method of Innis et al. (PCR Protocols. A Guide to Methods and Applications, 1990, Academic Press) with Pwo-Polymerase from Roche Diagnostics GmbH (Mannheim, Germany). With the aid of the polymerase chain reaction, the primers allow amplification of a DNA fragment 520 bp in size, which carries the incomplete tmk gene, including the native ribosome binding site.

The tmk fragment 520 bp in size was cleaved with the restriction endonucleases KpnI and XbaI and then isolated from the agarose gel with the QiaExII Gel Extraction Kit (Product No. 20021, Qiagen, Hilden, Germany).

### 3.2 Construction of the expression vector pXK99E

The IPTG-inducible expression vector pXK99E was constructed according to the prior art. The vector is based on the Escherichia coli expression vector pTRC99A (Amann et al., Gene 69: 301-315 (1988)) and contains the trc promoter, which can be induced by addition of the lactose derivative IPTG (isopropyl β-D-thiogalactopyranoside), the termination regions T1 and T2, the replication origin ColE1 from E. Coli, the lacI^{q} gene (repressor of the lac operon from E. coli), a multiple cloning site (mcs) (Norrander, J.M. et al. Gene 26, 101-106 (1983)) and the kanamycin resistance gene aph(3')-IIa from E. coli (Beck et al. (1982), Gene 19: 327-336).

It has been found the the vector pXK99E is quite specifically suitable for regulating the expression of a gene, in particular effecting attenuated expression in coryneform bacteria. The vector pXK99E is an E. coli expression vector and can be employed in E. coli for enhanced expression of a gene.

Since the vector cannot replicate independently in coryneform bacteria, this is retained in the cell only if it is integrated into the chromosome. The peculiarity of this vector here is the use for regulated expresion of a gene after cloning of a gene section from the front region of the corresponding gene in the vector containing the start codon and the native ribosome binding site, and subsequent integration of the vector into coryneform bacteria, in particular C. glutamicum. Gene expression is regulated by addition of metered amounts of IPTG to the nutrient medium. Amounts of 1 µM/l up to 10 µM/l IPTG have the effect of very weak expression of the corresponding gene, and amounts of 10 µM/l up to 100 µM/l have the effect of a slightly attenuated to normal expression of the corresponding gene.

The E. coli expression vector pXK99E constructed was transferred by means of electroporation (Tauch et al. 1994, FEMS Microbiol Letters, 123: 343-347) into E. coli DH5αmcr (Grant, 1990, Proceedings of the National Academy of Sciences U.S.A., 87:4645-4649). Selection of the transformants was carried out on LB Agar (Sambrook et al., Molecular Cloning: A Laboratory Manual. 2^{nd} Ed., Cold Spring Harbor Laboratory Press, Cold Spring Harbor, N.Y., 1989), which had been supplemented with 50 mg/l kanamycin.

Plasmid DNA was isolated from a transformant by conventional methods (Peters-Wendisch et al., 1998, Microbiology, 144, 915 - 927), cleaved with the restriction endonuclease NcoI, and the plasmid was checked by subsequent agarose gel electrophoresis.

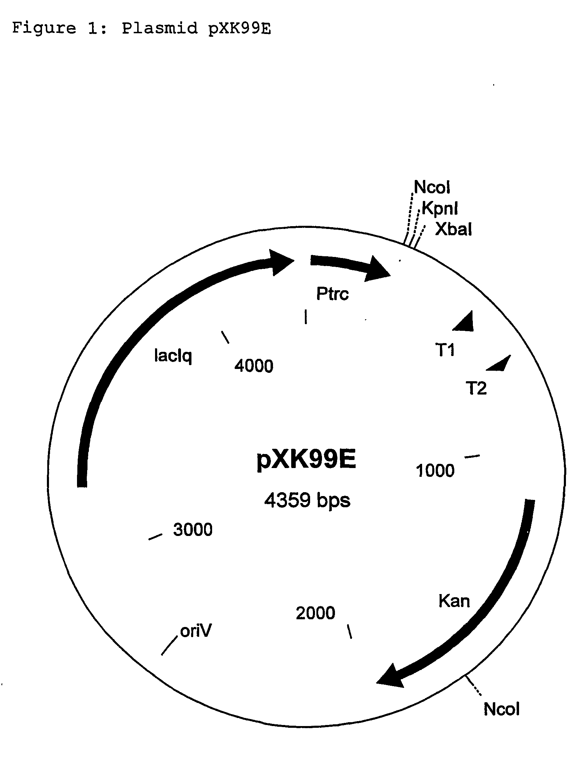

The plasmid construct obtained in this way was called pXK99E (figure 1). The strain obtained by electroporation of the plasmid pXK99E in the E.coli strain DH5αmcr was called E.coli DH5alphamcr/pXK99E (= DH5αmcr/pXK99E) and deposited on 31st July 2001 as DSM 14440 at the Deutsche Sammlung für Mikroorganismen und Zellkulturen (DSMZ = German Collection of Microorganisms and Cell Cultures, Braunschweig, Germany) in accordance with the Budapest Treaty.

### 3.3 Cloning of the tmk fragment in the E. coli expression vector pXK99E

The E. coli expression vector pXK99E described in example 3.2 was used as the vector. DNA of this plasmid was cleaved completely with the restriction enzymes KpnI and XbaI and then dephosphorylated with shrimp alkaline phosphatase (Roche Diagnostics GmbH, Mannheim, Germany, Product Description SAP, Product No. 1758250).

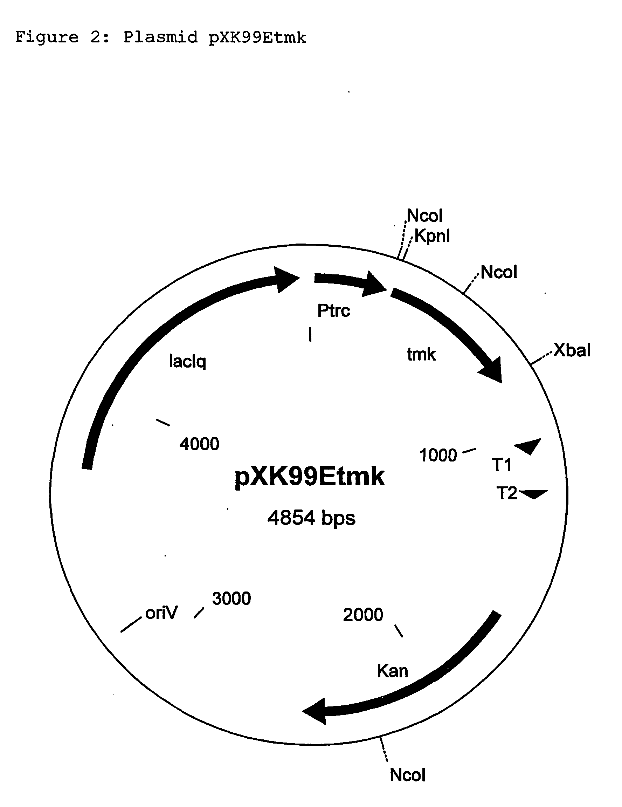

The tmk fragment approx. 500 bp in size described in example 3.1, obtained by means of PCR and cleaved with the restriction endonucleases KpnI and XbaI was mixed with the prepared vector pXK99E and the batch was treated with T4 DNA ligase (Amersham Pharmacia, Freiburg, Germany, Product Description T4-DNA-Ligase, Code no.27-0870-04). The ligation batch was transformed in the E. coli strain DH5αmcr (Hanahan, In: DNA cloning. A Practical Approach. Vol. I, IRL-Press, Oxford, Washington DC, USA). Selection of plasmid-carrying cells was made by plating out the transformation batch on LB agar (Lennox, 1955, Virology, 1:190) with 50 mg/l kanamycin. After incubation overnight at 37°C, recombinant individual clones were selected. Plasmid DNA was isolated from a transformant with the Qiaprep Spin Miniprep Kit (Product No. 27106, Qiagen, Hilden, Germany) in accordance with the manufacturer's instructions and cleaved with the restriction enzymes KpnI and XbaI to check the plasmid by subsequent agarose gel electrophoresis. The resulting plasmid was called pXK99Etmk. It is shown in figure 2.

### Example 4

### Integration of the vector pXK99Etmk into the genome of the C. glutamicum strain DSM5715

The vector pXK99E mentioned in example 3 was elecroporated [sic] by the elecroporation [sic] method of Tauch et al.,(1989 FEMS Microbiology Letters 123: 343-347) in the strain C. glutamicum DSM5715. The vector cannot replicate independently in DSM5715 and is retained in the cell only if it has integrated into the chromosome. Selection of clones with integrated pXK99Etmk was carried out by plating out the elecroporation [sic] batch on LB agar (Sambrock [sic] et al., Molecular Cloning: A Laboratory Manual. 2^{nd} Ed., Cold Spring Harbor, New York, 1989), which had been supplemented with 15 mg/l kanamycin and IPTG (1mM/l).

For detection of the integration, the tmk fragment was labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes NcoI and KpnI. The fragments formed were separated by means of agarose gel electrophoresis and hybridized at 68°C with the Dig hybrization [sic] kit from Boehringer. The plasmid pXK99Etmk mentioned in example 3 had been inserted into the chromosome of DSM5715 within the chromosomal tmk gene. The strain was called DSM5715::pXK99Etmk.

### Example 5

### Preparation of lysine

The C. glutamicum strain DSM5715::pXK99Etmk obtained in example 4 was cultured in a nutrient medium suitable for the production of lysine and the lysine content in the culture supernatant was determined. By addition of IPTG (10 µM/l), attenuated expression of the tmk gene occurs, regulated by the trc promoter.

For this, the strain was first incubated on an agar plate with the corresponding antibiotic (brain-heart agar with kanamycin (25 mg/l) and IPTG (10 µM/l)) for 24 hours at 33ºC. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Kanamycin (25 mg/l) and IPTG (10 µM/l) were added to this. The preculture was incubated for 16 hours at 33ºC at 240 rpm on a shaking machine. A main culture was seeded from this preculture such that the initial OD (660 nm) of the main culture was 0.1 OD. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 50g/l |

| Salts: | |
|---|---|
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| Leucine (sterile-filtered) | 0.1 g/l |
| CaCO₃ | 25 g/l |

The CSL, MOPS and the salt solution are brought to pH 7 with aqueous ammonia and autoclaved. The sterile substrate and vitamin solutions are then added, and the CaCO₃ autoclaved in the dry state is added.

Culturing is carried out in a 10 ml volume in a 100 ml conical flask with baffles. Kanamycin (25 mg/l) and IPTG (10 µM/l) were added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in table 1.

**Table 1**

| Strain | OD (660 nm) | Lysine HCl g/l |
|---|---|---|
| DSM5715 | 7.8 | 13.58 |
| DSM5715::pXK99Etmk | 11.3 | 15.51 |

### Brief description of the figures:

- Figure 1:: Map of the plasmid pXK99E,
- Figure 2:: Map of the plasmid pXK99Etmk.

The abbreviations and designations used have the following meaning.
- Kan:: Kanamycin resistance gene aph(3')-IIa from Escherichia coli
- KpnI: Cleavage site of the restriction enzyme KpnI
- NcoI: Cleavage site of the restriction enzyme NcoI
- XbaI: Cleavage site of the restriction enzyme XbaI
- Ptrc: trc promoter
- T1: Termination region T1
- T2: Termination region T2
- lacIq: lacIq repressor of the lac operon of Escherichia coli
- oriV: Replikation origin ColE1 from E. coli
- tmk: Cloned region of the tmk gene

## Claims

1. Isolated coryneform bacteria wherein the activity or concentration of the protein having an amino acid sequence which has at least 90 % identity to the amino acid sequence set out in SEQ ID NO:2 and has thymidylate kinase activity is reduced to 0 % of the activity or concentration of said protein of the starting microorganism.

2. Coryneform bacteria according to claim 1, wherein the amino acid sequence of said protein is set out in SEQ ID NO:2.

3. Isolated coryneform bacteria wherein the activity or concentration of the protein encoded by the polynucleotide set out in SEQ ID NO:1 is reduced to 0 % of the activity or concentration of the activity or concentration of the protein of the starting microorganism.

4. Strains of the species Corynebacterium glutamicum according to one or more of the claims 1 to 3, wherein the activity or concentration of said protein is reduced to 0 %.

5. Escherichia coli DH5alphamcr/pXK99Etmk (= DH5αmcr/pXK99Etmk), deposited under DSM 14439 at the Deutsche Sammlung für Mikroorganismen und Zellkulturen [German Collection of Microorganisms and Cell Cultures], DSMZ, Braunschweig, Germany.

6. Process for the fermentative preparation of L-lysine, comprising:
fermentation of the coryneform bacteria according to one or more of the claims 1 to 4 which produce said L-amino acid

7. The process according to claim 7, comprising
fermentation of said bacteria
concentration of said amino acid, in the medium or in the cells of the bacteria, and
isolation of said amino acid.

8. Process according to the claims 7 or 8, wherein bacteria are employed, in which the concentration or activity of proteins encoded by further genes of the biosynthesis pathway of L-lysine are increased by 10 to 2000 %.

9. Process according to claims 7 or 8, wherein bacteria are employed in which the concentration or activity of proteins encoded by genes of the metabolic pathways which reduce the formation of L-lysine acid are reduced to 0 %.

10. Process according to claim 7, wherein the expression of the polynucleotide(s) which code(s) for the thymidylate kinase gene which sequence is set out in , SEQ ID NO:1 is eliminated by transition, transversion, insertion or deletion.

11. Process according to claim 9, wherein for the preparation of L-lysine, coryneform microorganisms are fermented in which at the same time the activity or concentration of one ore more of the proteins encoded by the genes chosen from the group consisting of
the dapA gene which codes for dihydrodipicolinate synthase,
the gap gene which codes for glyceraldehyde 3-phosphate dehydrogenase,
the tpi gene which codes for triose phosphate isomerase,
the pgk gene which codes for 3-phosphoglycerate kinase,
the zwf gene which codes for glucose 6-phosphate dehydrogenase,
the pyc gene which codes for pyruvate carboxylase,
the mqo gene which codes for malate-quinone oxidoreductase,
the lysC gene which codes for a feed-back resistant aspartate kinase,
the lysE gene which codes for lysine export,
the zwa1 gene which codes for the Zwa1 protein
is or are increased by 10 to 2000 %.

12. Process according to claim 10, wherein for the preparation of L-lysine, coryneform microorganisms are fermented in which at the same time the activity or concentration of one or more of the proteins encoded by the genes chosen from the group consisting of
the pck gene which codes for phosphoenol pyruvate carboxykinase,
the pgi gene which codes for glucose 6-phosphate isomerase,
the poxB gene which codes for pyruvate oxidase;
the zwa2 gene which codes for the Zwa2 protein,
is or are reduced to 0 %.

13. Process according to one or more of claims 5 - 13, wherein microorganisms of the species Corynebacterium glutamicum are employed.

14. Process according to claim 11, wherein the Corynebacterium strain DSM5715::pXK99Etmk is employed.

15. Vector pXK99Etmk,
the restriction map of which is reproduced in figure 2, and
which is deposited in the E. coli strain DH5alphamcr/pXK99Etmk under no. DSM 14439 at the Deutsche Sammlung für Mikroorganismen und Zellkulturen [German Collection of Microorganisms and Cell Cultures].

## Patentansprüche

1. Isolierte coryneforme Bakterien, wobei die Aktivität oder Konzentration des Proteins mit einer Aminosäuresequenz, die mindestens 90% Identität mit der unter SEQ ID NO:2 aufgeführten Aminosäuresequenz sowie Thymidylatkinaseaktivität aufweist, auf 0% der Aktivität oder Konzentration des Proteins des Ausgangsmikroorganismus reduziert ist.

2. Coryneforme Bakterien gemäß Anspruch 1, wobei die Aminosäuresequenz des Proteins unter SEQ ID NO:2 aufgeführt ist.

3. Isolierte coryneforme Bakterien, wobei die Aktivität oder Konzentration des von dem unter SEQ ID NO:1 aufgeführten Polynukleotid codierten Proteins auf 0% der Aktivität oder Konzentration der Aktivität oder Konzentration des Proteins des Ausgangsmikroorganismus reduziert ist.

4. Stämme der Spezies Corynebacterium glutamicum gemäß einem oder mehreren der Ansprüche 1 bis 3, wobei die Aktivität oder Konzentration des Proteins auf 0% reduziert ist.

5. Escherichia coli DH5alphamcr/pXK99Etmk (= DH5αmcr/pXK99Etmk), hinterlegt unter DSM 14439 bei der Deutschen Sammlung für Mikroorganismen und Zellkulturen (DSMZ), Braunschweig.

6. Verfahren zur fermentativen Herstellung von L-Lysin, umfassend:
Fermentation der diese L-Aminosäure produzierenden coryneformen Bakterien gemäß einem oder mehreren der Ansprüche 1 bis 4.

7. Verfahren gemäß Anspruch 7, umfassend:
Fermentation der Bakterien,
Konzentration der Aminosäure entweder im Medium oder in den Zellen der Bakterien und
Isolierung der Aminosäure.

8. Verfahren gemäß Anspruch 7 oder 8, wobei man Bakterien einsetzt, in denen die Konzentration oder Aktivität von von weiteren Genen des Biosynthesewegs von L-Lysin codierten Proteinen um 10 bis 2000% erhöht ist.

9. Verfahren gemäß Anspruch 7 oder 8, wobei man Bakterien einsetzt, in denen die Konzentration oder Aktivität von von Genen derjenigen Stoffwechselwege, durch die die Bildung von L-Lysin herabgesetzt wird, codierten Proteinen auf 0% reduziert ist.

10. Verfahren gemäß Anspruch 7, wobei die Expression des bzw. der für das Thymidylatkinasegen, dessen Sequenz unter SEQ ID NO:1 aufgeführt ist, codierenden Polynukleotids bzw. Polynukleotide durch Transition, Transversion, Insertion oder Deletion ausgeschaltet wird.

11. Verfahren gemäß Anspruch 9, wobei zur Herstellung von L-Lysin coryneforme Mikroorganismen fermentiert werden, in denen gleichzeitg die Aktivität oder Konzentration eines oder mehrerer der von den Genen, gewählt aus der Gruppe bestehend aus:
dem für Dihydrodipicolinat-Synthase codierenden Gen dapA,
dem für Glycerinaldehyd-3-phosphat-Dehydrogenase codierenden Gen gap,
dem für Triosephosphat-Isomerase codierenden Gen tpi,
dem für 3-Phosphoglycerat-Kinase codierenden Gen pgk,
dem für Glucose-6-phosphat-Dehydrogenase codierenden Gen zwf,
dem für Pyruvat-Carboxylase codierenden Gen pyc,
dem für Malat-Chinon-Oxidoredukase codierenden Gen mqo,
dem für eine feedbackresistente Aspartatkinase codierenden Gen lysC,
dem für den Lysin-Export codierenden Gen lysE,
dem für das Protein Zwa1 codierenden Gen zwa1,
codierten Proteine um 10 bis 2000% erhöht ist oder sind.

12. Verfahren gemäß Anspruch 10, wobei zur Herstellung von L-Lysin coryneforme Mikroorganismen fermentiert werden, in denen gleichzeitg die Aktivität oder Konzentration eines oder mehrerer der von den Genen, gewählt aus der Gruppe bestehend aus:
dem für Phosphoenolpyruvat-Carboxykinase codierenden Gen pck,
dem für Glucose-6-phosphat-Isomerase codierenden Gen pgi,
dem für Pyruvatoxidase codierenden Gen poxB,
dem für das Protein Zwa2 codierenden Gen zwa2,
codierten Proteine auf 0% reduziert ist oder sind.

13. Verfahren gemäß einem oder mehreren der Ansprüche 5 - 13, wobei Mikroorganismen der Spezies Corynebacterium glutamicum eingesetzt werden.

14. Verfahren gemäß Anspruch 11, wobei der Corynebacterium-Stamm DSM5715::pXK99Etmk eingesetzt wird.

15. Vektor pXK99Etmk,
dessen Restriktionskarte in Figur 2 wiedergegeben ist und
der im E.-coli-Stamm DH5alphamcr/pXK99Etmk unter Nr. DSM 14439 bei der Deutschen Sammlung für Mikroorganismen und Zellkulturen hinterlegt ist.

## Revendications

1. Bactérie isolée du genre Corynebacterium dans laquelle l'activité ou la concentration de la protéine présentant une séquence d'acides aminés qui est identique au moins à 90% à la séquence d'acides aminés décrite à la SEQ ID NO:2 et qui présente une activité de thymidylate kinase est réduite à 0% de l'activité ou de la concentration de ladite protéine du microorganisme de départ.

2. Bactérie du genre Corynebacterium suivant la revendication 1, dans laquelle la séquence d'acides aminés de ladite protéine est décrite à la SEQ ID NO:2.

3. Bactérie isolée du genre Corynebacterium dans laquelle l'activité ou la concentration de la protéine codée par le polynucléotide décrit à la SEQ ID NO:1 est réduite à 0% de l'activité ou de la concentration de la protéine du microorganisme de départ.

4. Souche de l'espèce Corynebacterium glutamicum suivant une ou plusieurs des revendications 1 à 3, dans laquelle l'activité ou la concentration de ladite protéine est réduite à 0%

5. Escherichia coli DH5alphamcr/pXK99Etmk (=DH5αmcr/pXK99Etmk), déposée sous la référence DSM 14 439 à la Deutsche Sammlung für Mikroorganismen und Zellkulturen [Collection allemande de microorganismes et de cultures cellulaires], DSMZ, Braunschweig, Allemagne.

6. Procédé pour la préparation par fermentation de L-lysine comprenant :
une fermentation de la bactérie du genre Corynebacterium suivant une ou plusieurs des revendications 1 à 4 qui produit ledit acide aminé L.

7. Procédé suivant la revendication 7, comprenant
une fermentation de ladite bactérie
une concentration dudit acide aminé, dans le milieu ou dans les cellules des bactéries, et
un isolement dudit acide aminé.

8. Procédé suivant la revendication 7 ou 8, dans lequel on utilise des bactéries, dans lesquelles la concentration ou l'activité de protéines codées par d'autres gènes de la voie de biosynthèse de la L-lysine est augmentée de 10 à 2000%.

9. Procédé suivant la revendication 7 ou 8, dans lequel on utilise des bactéries dans lesquelles la concentration ou l'activité de protéines codées par des gènes de voies métaboliques qui réduisent la formation de l'acide de la L-lysine est réduite à 0%.

10. Procédé suivant la revendication 7, dans lequel l'expression du(des) polynucléotide(s) qui code(nt) pour le gène de la thymidylate kinase dont la séquence est décrite à la SEQ ID NO:1 est éliminée par une transition, une transversion, une insertion ou une délétion.

11. Procédé suivant la revendication 9, dans lequel pour la préparation de L-lysine, on met en fermentation des microorganismes du genre Corynebacterium dans lesquels en même temps, est accrue de 10 à 2000% l'activité ou la concentration d'une ou de plusieurs des protéines codées par les gènes choisis parmi le groupe comprenant :
le gène dapA qui code pour une dihydrodipicolinate synthase,
le gène gap qui code pour une glycéraldéhyde-3-phosphate déshydrogénase,
le gène tpi qui code pour une triose-phosphate isomérase,
le gène pgk qui code pour une 3-phosphoglycérate kinase,
le gène zwf qui code pour une glucose-6-phosphate déshydrogénase,
le gène pyc qui code pour une pyruvate carboxylase,
le gène mqo qui code pour une malate-quinone oxydoréductase,
le gène lysC qui code pour une aspartate kinase résistante à une rétroaction,
le gène lysE qui code pour une fonction d'export de la lysine,
le gène zwa1 qui code pour la protéine zwa1.

12. Procédé suivant la revendication 10, dans lequel pour la préparation de L-lysine, on met en fermentation des microorganismes du genre Corynebacterium dans lesquels en même temps, est réduite à 0% l'activité ou la concentration d'une ou de plusieurs des protéines codées par les gènes choisis parmi le groupe comprenant le gène pck qui code pour une phosphoénolpyruvate carboxykinase,
le gène pgi qui code pour une glucose-6-phosphate isomérase,
le gène poxB qui code pour une pyruvate oxydase,
le gène zwa2 qui code pour la protéine Zwa2.

13. Procédé suivant une ou plusieurs des revendications 5-13, dans lequel on utilise des microorganismes de l'espèce Corynebacterium glutamicum.

14. Procédé suivant la revendication 11, dans lequel on utilise la souche de Corynebacterium DSM5715::pXK99Etmk.

15. Vecteur pXK99Etmk, dont la cartographie de restriction est reproduite à la Fig. 2, et qui est déposé dans la souche de E.coli DH5alphamcr/pXK99Etmk sous le n° DSM 14 439 à la Deutsche Sammlung für Mikroorganismen und Zellkulturen [Collection allemande de microorganismes et de cultures cellulaires].


### SEQUENCE PROTOCOL

<110> Degussa AG
<120> Nucleotide sequences which code for the tmk gene
<130> 000481 BT
<140>
   <141>
<160> 4
<170> PatentIn Ver. 2.1
<210> 1
   <211> 1120
   <212> DNA
   <213> Corynebacterium glutamicum
<220>
   <221> CDS
   <222> (244)..(852)
   <223> tmk gene
<400> 1
   acacccatca tttgtcatgt ccaactcttt cgccgatcag accattgcgc agatcgaact 60
   gttccaaaac gaaggacagt acgagaacga ggtctaccgt ctgcctaagg ttctcgacga 120
   aaaggtggca cgcatccacg ttgaggctct cggcggtcag ctcaccgaac tgaccaagga 180
   gcaggctgag tacatcggcg ttgacgttgc aggcccattc aagccggagc actaccgcta 240
   cta atg att gtc agc att gag gga atc gac ggc gcc ggc aaa aac acc    288
       Met Ile Val Ser Ile Glu Gly Ile Asp Gly Ala Gly Lys Asn Thr
         1               5                  10                  15
   ctg gtt tcg gca tta acg cag gtt att gat gca aaa gtc ctt gca ttc    336
   Leu Val Ser Ala Leu Thr Gln Val Ile Asp Ala Lys Val Leu Ala Phe
                    20                  25                  30
   cca cgt tat gaa acc tcg att cac gcc caa ttg gcc gcg gaa gca ctc    384
   Pro Arg Tyr Glu Thr Ser Ile His Ala Gln Leu Ala Ala Glu Ala Leu
                35                  40                  45
   cac ggc cgc atg ggc gac ctc acc gac agc gcc tac gcc atg gcc acg    432
   His Gly Arg Met Gly Asp Leu Thr Asp Ser Ala Tyr Ala Met Ala Thr
            50                  55                  60
   ctt ttc gcc ctc gac cgc cac ttc gcg att gat gac tta aat gcg ccc    480
   Leu Phe Ala Leu Asp Arg His Phe Ala Ile Asp Asp Leu Asn Ala Pro
        65                  70                  75
   ggc gtg gtg ctg ctc gac cga tac gtc gcc tcc aac gcg gct tat acc    528
   Gly Val Val Leu Leu Asp Arg Tyr Val Ala Ser Asn Ala Ala Tyr Thr
    80                  85                  90                  95
   gcc gcc aga ttg ctt gac gac gac gcc ccc cgc tgg gtt gcc gac ctg    576
   Ala Ala Arg Leu Leu Asp Asp Asp Ala Pro Arg Trp Val Ala Asp Leu
                   100                 105                 110
   gaa ttc ggg cgg ctt ggg ctc cca cgt ccg acg ctt caa gtg ttg ttg    624
   Glu Phe Gly Arg Leu Gly Leu Pro Arg Pro Thr Leu Gln Val Leu Leu
               115                 120                 125
   gat acc ccc gcg gag gta gcg caa gat agg gct aga cgt cga gaa gcg    672
   Asp Thr Pro Ala Glu Val Ala Gln Asp Arg Ala Arg Arg Arg Glu Ala
           130                 135                 140
   ctt gac tcc gcg cgt gcg cgg gac cgc tat gaa tcg gat tcg gcg ctg    720
   Leu Asp Ser Ala Arg Ala Arg Asp Arg Tyr Glu Ser Asp Ser Ala Leu
       145                 150                 155
   cag caa cgc acc gcc gag cac tat cgc cgc ctc gcg gcg gac aac tgg    768
   Gln Gln Arg Thr Ala Glu His Tyr Arg Arg Leu Ala Ala Asp Asn Trp
   160                 165                 170                 175
   gaa tca ccg tgg atc gtg gtt gcc cct gat gaa gac ccc ggc cac gtt    816
   Glu Ser Pro Trp Ile Val Val Ala Pro Asp Glu Asp Pro Gly His Val
                   180                 185                 190
   gcg cag aga atc gtg gaa ttc ctg ggt act ata aac taatcccaat         862
   Ala Gln Arg Ile Val Glu Phe Leu Gly Thr Ile Asn
               195                 200
   tagcaggaag gattctcatg tcacagaaaa ttctcgtggt tgatgatgat cccgccatct 922
   ccgagatgct caccatcgtg ctcagcgcag aaggctttga caccgtagct gtcaccgacg 982
   gcgcactcgc cgtggaaacc gcctcccggg aacaaccgga tctgattttg ctcgacttga 1042
   tgcttccagg catgaacggc atcgacattt gtcgcctcat ccgccaagaa tcctccgtac 1102
   ccatcatcat gctcaccg                                               1120
<210> 2
   <211> 203
   <212> PRT
   <213> Corynebacterium glutamicum
<400> 2
   Met Ile Val Ser Ile Glu Gly Ile Asp Gly Ala Gly Lys Asn Thr Leu
   1               5                   10                  15
   Val Ser Ala Leu Thr Gln Val Ile Asp Ala Lys Val Leu Ala Phe Pro
               20                  25                  30
   Arg Tyr Glu Thr Ser Ile His Ala Gln Leu Ala Ala Glu Ala Leu His
           35                  40                  45
   Gly Arg Met Gly Asp Leu Thr Asp Ser Ala Tyr Ala Met Ala Thr Leu
       50                  55                  60
   Phe Ala Leu Asp Arg His Phe Ala Ile Asp Asp Leu Asn Ala Pro Gly
   65                  70                  75                  80
   Val Val Leu Leu Asp Arg Tyr Val Ala Ser Asn Ala Ala Tyr Thr Ala
                   85                  90                  95
   Ala Arg Leu Leu Asp Asp Asp Ala Pro Arg Trp Val Ala Asp Leu Glu
               100                 105                 110
   Phe Gly Arg Leu Gly Leu Pro Arg Pro Thr Leu Gln Val Leu Leu Asp
           115                 120                 125
   Thr Pro Ala Glu Val Ala Gln Asp Arg Ala Arg Arg Arg Glu Ala Leu
       130                 135                 140
   Asp Ser Ala Arg Ala Arg Asp Arg Tyr Glu Ser Asp Ser Ala Leu Gln
   145                 150                 155                 160
   Gln Arg Thr Ala Glu His Tyr Arg Arg Leu Ala Ala Asp Asn Trp Glu
                   165                 170                 175
   Ser Pro Trp Ile Val Val Ala Pro Asp Glu Asp Pro Gly His Val Ala
               180                 185                 190
   Gln Arg Ile Val Glu Phe Leu Gly Thr Ile Asn
           195                 200
<210> 3
   <211> 28
   <212> DNA
   <213> Artificial sequence
<220>
   <223> Description of the artificial sequence: Primer tmk for
<400> 3
   tgggtaccat tcaagccgga gcactacc                                  28
<210> 4
   <211> 28
   <212> DNA
   <213> Artificial sequence
<220>
   <223> Description of the artificial sequence: Primer tmk int
<400> 4
   gatctagaca gcgccgaatc cgattcat                                        28